Europäisches Patentamt

European Patent Office

Office européen des brevets

(19)

(11) Publication number: **0 195 516**

**A1**

(12) **EUROPEAN PATENT APPLICATION**

(21) Application number: **86301098.9**

(22) Date of filing: **18.02.86**

(51) Int. Cl.⁴: **C 07 C 15/02**

(30) Priority: **22.03.85 GB 8507519**

(43) Date of publication of application:
**24.09.86 Bulletin 86/39**

(84) Designated Contracting States:
**DE FR NL**

(71) Applicant: **Coal Industry (Patents) Limited**
**Hobart House Grosvenor Place**
**London SW1X 7AE(GB)**

(72) Inventor: **Robinson, Joseph Gordon**
**Claybrook The Hyde**
**Winchcombe Gloucestershire(GB)**

(72) Inventor: **Barnes, Ian David**
**78 Swindon Lane Prestbury**
**Cheltenham Gloucestershire(GB)**

(72) Inventor: **Riemer, Pierce William Foster**
**27 Lambourne Avenue**
**Huntley Gloucestershire(GB)**

(74) Representative: **Wood, John Irwin et al,**
**Hobart House Grosvenor Place**
**London SW1X 7AE(GB)**

(54) **Improvements in hydrocarbon processing.**

(57) A mixture of C10 to C14 aromatic hydrocarbons, e.g., resulting from a synthesis reaction, is upgraded to lower alkyl benzenes by passing the mixture over an aluminosilicate catalyst together with a catalytic additive, which can be recovered for re-use. At least an equivalent weight of catalytic additive is used and the upgrading is carried out in the vapour phase.

## IMPROVEMENTS IN HYDROCARBON PROCESSING

This invention concerns improvments in hydrocarbon processing, more particularly it concerns the upgrading of aromatic hydrocarbon mixtures.

In our UK Patent No's 2,100,710 and 2,132,595 we describe modified amorphous silica - based catalysts which are effective in the conversion of oxygen-containing aliphatics into hydrocarbons. Such an activity is also known to take place over crystalline zeolite-type catalysts, especially the synthetic crystalline zeolite ZSM-5, and modifications thereof. Catalysts made according to the aforementioned UK Patents exhibit, under preferred conditions, a high specificity towards aromatic hydrocarbons, up to C13 or C14 aromatics, with considerable quantities of C10, C11 and C12 aromatics. These aromatics are alkyl benzenes.

The higher alkyl benzenes are less valuable than the lower alkyl benzenes and it is an aim of the present invention to upgrade these higher aromatic hydrocarbons of C10 to C14, whether they have resulted from a synthesis reaction of the kind described, or not.

The present invention provides a method of upgrading C10 to C14 alkyl benzenes comprising passing a vapour phase feedstock containing a C10 to C14 alkylbenzene at elevated temperature over a microporous aluminosilicate catalyst in the presence of at least an equivalent amount by weight of the alkylbenzene of a catalytic additive which is toluene or a xylene, and wherein at least 90% by weight of the catalytic additive is recoverable from the products.

In the method of the invention the feedstock will usually be a mixture of hydrocarbons, typically consisting of benzene (C6) and some or all of the intermediates up to C14 alkylbenzenes. The feedstock may contain other components, such as aliphatic hydrocarbons, or non-hydrocarbon compounds providing that such other components do not adversely interfere with the upgrading or contribute significantly to any catalyst deactivation.

Experimentally, it has been found that under preferred conditions, 100% of the catalytic additive is recovered from the product. This surprising result justifies the additive being termed a catalytic additive. The additive may, therefore, be used in a continuous re-cycle process.

The feedstock is passed at elevated temperature and in the vapour phase over the aluminosilicate catalyst; preferred temperatures are between 250 and 550°C, especially in the range from 400 to 500°C. The method may be carried out at elevated, reduced, or atmospheric pressure and it is convenient to use atmospheric pressure. The flow rate of feedstock plus catalytic additive, conveniently measured as a liquid hourly space velocity in experimental work in which a liquid hydrocarbon feed is vapourised

3

**0195516**

before passing it over the aluminosilicate catalyst, may vary from about 0.01 to 10/h, and preferably is in the range 0.3 to 5/h, especially 0.5 to 1.5/h.

The mixture of alkylbenzenes and catalytic additive may be premixed as liquids or in the vapour phase. The catalytic additive should be present in at least an equivalent amount by weight up to 10:1 and preferably at a ratio (additive: alkylbenzene) of at least 2:1, more preferably about 3:1.

The preferred aluminosilicate catalyst is an amorphous catalyst made according to the teaching of our UK Patents No.'s 2,100,710 and 2,132,595, more especially a protonated form such as "Catalyst B" of Example 1 of Patent No. 2,132,595. The catalyst may be suitably used in a fixed bed, although moving or fluidised beds may also be used in the method of the invention.

The invention will now be described with reference to the following Example.

EXAMPLE

A liquid hydrocarbon feedstock which was the product of passing methanol over "catalyst B" of Example 1 of UK Patent No 2,132,595 was blended with 3 times its weight of toluene. Samples of the resulting mixture were pumped through a pre-heater packed with glass beads before the mixture vapour was passed through a tubular reactor packed with the Catalyst B. The pre-heater and reactor were immersed in a fluidised sand bath maintained at 450°C. The products from the reactor were collected in an ice-cooled trap, and analysed by conventional gas-liquid chromatography. After correction of the results by removing from consideration the quantity of toluene co-fed with the liquid hydrocarbon, the analysis of the feed and product is given in the Table below. Less than 2% by weight of the product was gas, and this has not been included. Liquid yields were approximately 98% by weight.

TABLE

| COMPONENT | | FEED | PRODUCT |
|---|---|---|---|
| | | (Wt.%) | (Wt.%) |
| Benzene | | 1.10 | 1.41 |
| Toluene | | 1.85 | 2.26 |
| C8 | Arenes | 2.50 | 2.10 |
| C9 | Arenes | 26.89 | 65.49 |
| C10 | Arenes | 28.58 | 23.59 |
| C11 | Arenes | 15.65 | 4.31 |
| C12 | Arenes | 18.36 | 0.47 |
| C13 | Arenes | 3.14 | 0.30 |
| C14+ | Arenes | 1.94 | 0.06 |
| Hydrocarbons total | | 100.01 | 99.99 |
| STOICHIOMETRY | | | |
| Total hydrogen found (moles) | | 10.45 | 10.06 |
| Total Carbon found (moles) | | 7.46 | 7.49 |
| Hydrogen/Carbon ratio | | | |
| (theoretical) | | 1.36 | 1.36 |
| Hydrogen/Carbon ratio (found) | | 1.40 | 1.34 |

The very significant shift in products to C9 alkylbenzenes from C11 to C12 alkylbenzenes is illustrated in the accompanying bar chart figure.

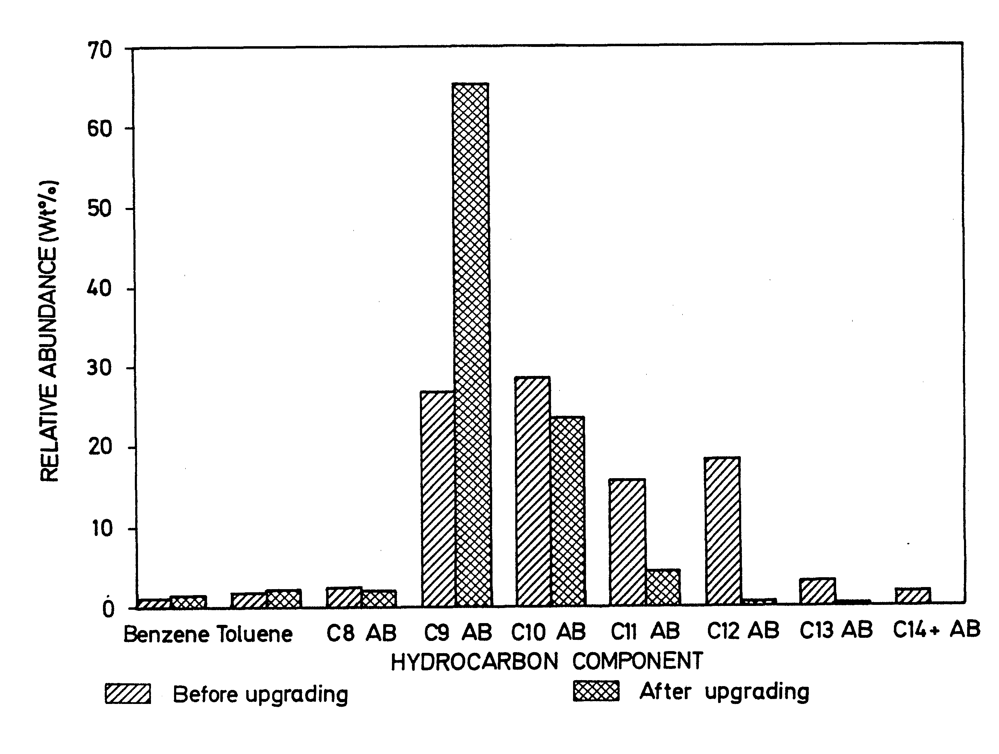

CLAIMS:

1. A method of upgrading C10 to C14 alkyl benzenes comprising passing a vapour phase feedstock containing a C10 to C14 alkyl benzene at elevated temperature over a microporous aliminosilicate catalyst in the presence of at least an equivalent amount by weight of the alkylbenzene of a catalytic additive which is toluene or xylene, under conditions wherein at least 90% by weight of the catalytic additive is recoverable from the products.

2. A method according to claim 1, wherein the additive is toluene.

3. A method according to claim 1 or 2, wherein the elevated temperature is between 250 and 550°C .

4. A method according to any one of claims 1 to 3, wherein the weight ratio of additive to feedstock is at least 2:1.

5. A method according to claim 4, wherein the weight ratio is about 3:1.

6. A method according to any one of the preceding claims, wherein the aluminosilicate catalyst is an amorphous catalyst according to UK Patent No. 2,100,710.

RELATIVE ABUNDANCE (Wt%) vs HYDROCARBON COMPONENT

Hydrocarbon components: Benzene, Toluene, C8 AB, C9 AB, C10 AB, C11 AB, C12 AB, C13 AB, C14+ AB

Legend: ▨ Before upgrading   ▨ After upgrading

0195516

# EUROPEAN SEARCH REPORT

European Patent Office

| | DOCUMENTS CONSIDERED TO BE RELEVANT | | | EP 86301098.9 |
|---|---|---|---|---|
| Category | Citation of document with indication, where appropriate, of relevant passages | | Relevant to claim | CLASSIFICATION OF THE APPLICATION (Int Cl 4) |
| A | EP - A1 - 0 082 702 (MOBIL OIL)<br>* Claims; examples * | | 1,3 | C 07 C 15/02 |
| A | FR - A1 - 2 471 359 (INSTITUT FRAN-CAIS DU PETROLE)<br>* Page 1, lines 1-28; claim 10 * | | 1-3 | |
| A | DE - A1 - 3 227 659 (UOP)<br>* Page 7, line 20 - page 10, line 24 * | | 1 | |
| A | US - A - 4 454 364 (FARCASIU et al.)<br>* Claims 7,12; example 1 * | | 1,3 | |
| A | DE - B2 - 1 643 154 (UCC)<br>* Claims; examples * | | 1,3 | TECHNICAL FIELDS SEARCHED (Int Cl 4) |
| A | DE - B - 2 040 556 (ASAHI KASEI KOGYO)<br>* Abstract; claims 1,3,4,5 * | | 1,3 | C 07 C 15/00<br>C 07 C 6/00<br>C 07 C 5/00<br>C 07 C 4/00 |
| D,A | GB - A - 2 132 595 (COAL INDUSTRY)<br>* Example 1 * | | 6 | |

The present search report has been drawn up for all claims

| Place of search | Date of completion of the search | Examiner |
|---|---|---|
| VIENNA | 25-04-1986 | KÖRBER |